# EUROPEAN PATENT APPLICATION

(11) **EP 4 374 900 A1**
(43) Date of publication of application: **29.05.2024**
(21) Application number: 22208794.2
(22) Date of filing: 22.11.2022
(51) Int. Cl.: A61M 16/08, A61M 16/20

(54) **RESPIRATION DEVICE AND SYSTEM FOR POSITIVE PRESSURE VENTILATION AND CONTINUOUS POSITIVE AIRWAY PRESSURE TREATMENT FOR NEONATAL RESUSCITATION**

(71) Applicant: Salk - Gemeinnützige Salzburger Landeskliniken Betriebsgesellschaft mbH, 5020 Salzburg (AT)
(72) Inventor: WALD, Martin, 5081 Anif (AT)
(74) Representative: Maiwald GmbH

(57) **Abstract**

A respiration device (10) for positive pressure ventilation (PPV) and continuous positive airway pressure (CPAP) treatment for neonatal resuscitation is described. The respiration device comprises: a body part (20) with a breathing gas inlet (22), a breathing gas outlet (24) in fluid communication with the breathing gas inlet and fluidly couplable to a patient interface (510), a first outflow port (26) for releasing breathing gas to the surrounding and a second outflow port (28) fluidly couplable to an expiration valve (32) or an expiratory branch (52) of a ventilation machine (210b); and a CPAP generator (30) fluidly coupled between the breathing gas inlet and the breathing gas outlet. The respiration device is operable either in a CPAP mode based on releasing breathing gas from the body part to the surrounding via the first outflow port, or in a PPV mode based on at least intermittently occluding the first outflow port.

## Description

### Technical Field

The present invention generally relates to the field of respiration devices and systems. In particular, the present invention relates to a respiration device and system for positive pressure ventilation (PPV) and continuous positive airway pressure (CPAP) treatment for neonatal resuscitation and/or initial respiratory support.

### Technical Background

A significant number of newborns do not start breathing on their own after birth but require respiratory support or artificial respiration (also referred to as mechanical ventilation). Typically, about 85% of newborns start breathing on their own, 10% need an additional stimulus and about 5% need respiratory support and/or mechanical ventilation. In particular, premature infants, for example newborns with a gestational age of less than 259, can suffer from respiratory distress and may require neonatal resuscitation.

In neonatal resuscitation, newborns who can breathe at least to a certain extent spontaneously or on its own are usually treated by continuous positive airway pressure ventilation (CPAP treatment or ventilation). This non-invasive ventilation uses a continuous low positive airway pressure (CPAP) to enable the lungs to be kept "open" by applying a positive end-expiratory pressure (PEEP). In CPAP ventilation, an oxygen-air mixture is usually introduced into the child's throat with slight overpressure so that a residual amount of air, respectively the mixture, remains in the lungs even during exhalation and prevents them from collapsing due to the positive end-expiratory pressure or permanent overpressure. Newborns who do not or only insufficiently breathe by themselves require respiratory support by means of positive pressure ventilation (PPV).

For positive pressure ventilation, for example in case of respiratory arrest, resuscitator bags or T-piece devices are typically used in the field of neonatal resuscitation, for example using a mask as interface to the newborn or an endotracheal intubation tube (ET tube). In case of mask ventilation, newborns should preferably be ventilated with a CPAP and/or PEEP according to established guidelines. Since a CPAP and/or PEEP cannot be maintained with a resuscitator bag, T-piece devices or ventilation systems have become widely used in clinical workflows for neonatal resuscitation and initial respiratory support.

Conventional T-pieces or T-piece devices comprise three openings, one of which receives fresh breathing gas, for example supplied from a flow or breathing gas supply (e.g. as oxygen-air mixture) of a first aid unit or resuscitator device. Another opening serves for delivering the breathing gas to the patient. This can be done either via an interface, such as a respiratory mask, which can be placed tightly over the patient's mouth and/or nose, or via a respiratory tube, an ET tube, through which the breathing gas can be fed directly into the patient's lungs. The third opening of a conventional T-piece can be designed as an outflow opening or valve to generate the PEEP and/or CPAP pressure. At rest, the entire gas can flow through this outflow opening into the environment, thereby creating the CPAP/PEEP pressure due to the outflow resistance throughout the tubing system and, accordingly, in the patient's lungs. When the patient is breathing adequately spontaneously, the inflowing fresh breathing gas is inhaled and used breathing gas is exhaled via the outflow opening, whereby the CPAP/PEEP pressure is maintained, this is the pressure does not drop to the ambient pressure, even at the end of expiration.

In the event of respiratory arrest and/or for PPV ventilation, the outflow opening on the T-piece can be occluded, for example with a finger. This causes the pressure in the entire tube system to rise rapidly, resulting in manual or mechanical inspiration and inflation of the lungs. To prevent overinflation and over-breathing and potentially causing damage to the patient's lungs, typically a pressure relief valve or expiration valve is used in neonatal resuscitator devices, which opens upon reaching a maximum pressure or release pressure. As soon as the finger releases the outflow opening on the T-piece, the pressure can drop back to the CPAP/PEEP level, resulting in exhalation.

The functionality and working principle of resuscitator devices employing T-pieces, or CPAP devices based on the so-called Benveniste valve, as further described below, contrasts with the functionality of a ventilation machine, also referred to as ventilator, used at hospitals for mechanical ventilation and/or PPV. Such ventilation machines usually have a closed hose or tube circuit, which can be divided into two sections or branches by a so-called Y-piece. One section or branch functions as the inspiratory branch for the supply of fresh breathing gas, while the second section or branch functions as the expiratory branch for the removal of at least partially used or exhaled breathing gas. The lower or common leg of the Y-piece can be coupled to an ET tube for PPV ventilation, for example. Ventilators or ventilation machines for infants have a continuous flow of breathing gas through the inspiratory branch to the Y-piece, into the lungs and back through the expiratory branch. At the end of the expiratory branch, usually an expiration valve is arranged, which can control the pressure or CPAP pressure throughout the tube system via the machine. An inspiration occurs when the expiration valve closes, which builds up the ventilation pressure or PPV inflating the lungs. When a maximum pressure or release pressure is reached, the valve opens again and the pressure is held for a short time and then falls back to the initial pressure or CPAP/PEEP pressure, which causes an expiration to occur.

Other exemplary devices for CPAP ventilation or treatment can be based on the Benveniste valve, which was designed purely as a non-invasive CPAP system for premature infants. An advantage of these specialized CPAP devices is a reduced work of breathing when compared to conventional T-pieces.

Generally, the use of specialized CPAP devices for CPAP ventilation or treatment has been recommended and established standard in the initial care of newborns for years. In particular T-piece devices that function via a single-tube system, as described above, are recommended. In some cases, however, patients or newborns do not adequately adapt after birth under pure CPAP/PEEP ventilation and require mechanical ventilation. Typically, the entire ventilation equipment must be switched in those scenarios, which can be challenging in the course of routine work at hospitals and can require additional personnel. Also, double equipment may often be necessary in the initial care of premature infants, including a ventilation machine and for example a specialized CPAP device or resuscitator device with T-piece.

For example, when switching to mechanical or PPV ventilation using a bag during neonatal resuscitation, the ventilation should always be maintained manually during resuscitation in the delivery room. As a result, the person actuating the bag may not be available for other activities and further actions of treatment may potentially be delayed. On the other hand, if ventilation is to be continued via a ventilator or ventilation machine, then such a ventilator must be activated and used instead of the T-piece device, which may not allow for manual intervention in ventilation, for example until switching back to the CPAP/PEEP device or resuscitator device with T-piece.

### Summary

Therefore, it may be desirable to provide for an improved respiration device and system for PPV and CPAP treatment for neonatal resuscitation and/or initial respiratory support, particularly allowing for both manually controlled ventilation as with a conventional T-piece and mechanical ventilation, for example controlled by a ventilation machine or ventilator.

This is achieved by the subject matter of the independent claims. Exemplary embodiments are incorporated in the dependent claims and the following description.

Aspects of the present disclosure relate to a respiration device for PPV and CPAP treatment for neonatal resuscitation and/or initial respiratory support and to a respiration system comprising such respiration device. Any disclosure presented herein with reference to the respiration device equally applies to the respiration system, and vice versa.

According to an aspect of the present disclosure, there is provided a respiration device for positive pressure ventilation, PPV, and continuous positive airway pressure CPAP treatment or ventilation for neonatal resuscitation and/or initial respiratory support. The respiration device comprises a body part, wherein the body part includes a) a breathing gas inlet fluidly couplable to and/or configured for being coupled to a breathing gas supply for receiving a flow of breathing gas, for example fresh breathing gas, such as an oxygen-air mixture, b) a breathing gas outlet in fluid communication with the breathing gas inlet and fluidly couplable to and/or configured for being coupled to a patient interface for supplying (fresh) breathing gas to a patient, c) a first outflow port for releasing breathing gas from the body part, the first outflow port being fluidly couplable to a surrounding or environment of the respiration device, and d) a second outflow port for releasing breathing gas from the body part, the second outflow port being fluidly couplable to one or both an expiration valve and an expiratory branch of a ventilation machine or ventilator, for example for mechanical ventilation. The respiration device further comprises at least one continuous positive airway pressure, CPAP, generator fluidly coupled between the breathing gas inlet and the breathing gas outlet, and configured to generate a positive end-expiratory pressure for CPAP treatment or ventilation. Therein, the respiration device is operable in a CPAP mode for CPAP treatment or ventilation based on releasing breathing gas from the body part to the surrounding or environment of the respiration device via the first outflow port, such that the positive end-expiratory pressure, PEEP, is generated within at least a part of the body part and/or at the breathing gas outlet. Further, the respiration device is operable in a positive pressure ventilation, PPV, mode for PPV treatment or ventilation based on at least intermittently occluding and/or closing the first outflow port, such that a ventilation pressure for PPV treatment is generated within the body part.

In particular by providing the body part with the breathing gas inlet, the breathing gas outlet and the first and second outflow ports, one outflow port being couplable to a surrounding of the respiration device and another one being couplable to a ventilation machine or ventilator, in particular an expiratory branch thereof, the respiration device according to the present disclosure can advantageously be used for both manually controlled ventilation for CPAP treatment and mechanical ventilation controlled by a ventilation machine or ventilator for PPV treatment.

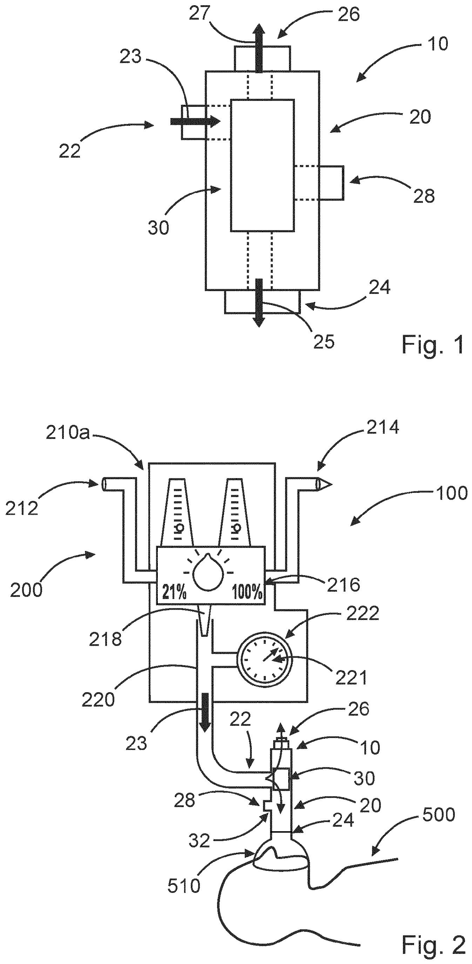

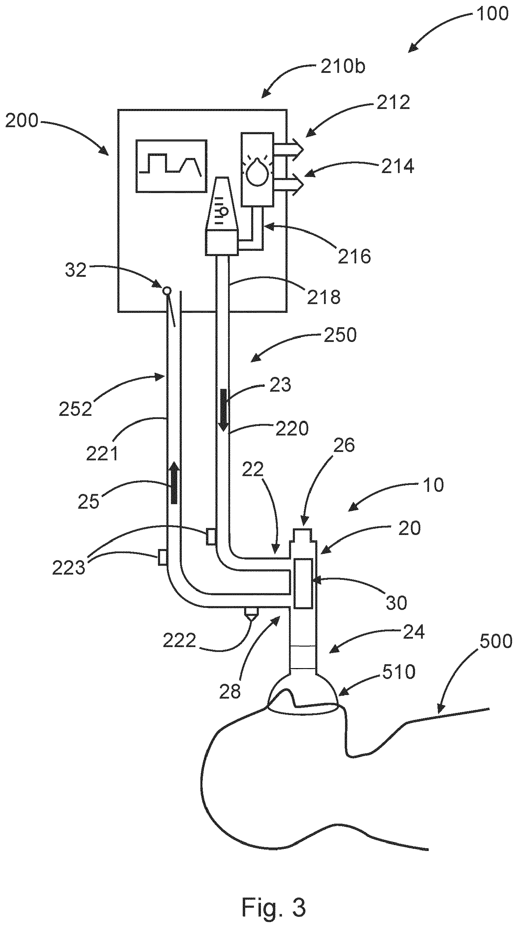

Also, the respiration device can be used with both single tube (or single hose) systems, as for example utilized with resuscitator devices and as exemplary described with reference to Figure 2, and double tube systems, as utilized for ventilation machines or ventilators and as exemplary described with reference to Figure 3. Accordingly, the respiration device of the present disclosure may combine the functionalities of conventional T-devices and Y-devices described above. Hence, versatility of the respiration device may be increased, and/or the need for switching between different ventilation devices or systems for CPAP and PPV treatment, for example when a patient or newborn does not adequately adapt after birth under pure CPAP/PEEP ventilation, can be eliminated. Also, it may be ensured that manual intervention of the ventilation, for example based on CPAP treatment, may always be possible by operating the respiration device in the CPAP mode.

For example, resuscitation can be started manually controlled in the CPAP mode and, if necessary, it can be switched to machine ventilation into the PPV mode, and vice versa, without changing the ventilation machine or other equipment, such as hoses and tubes. This may save time and personnel resources as well as eliminate the need for double equipment for CPAP and PPV treatment.

As used herein, the body part or body of the respiration device may refer to a support structure, for example a substantially solid support structure, at which the breathing gas inlet, the breathing gas outlet, the first outflow port, and the second outflow port may be arranged. At least a part of one or more of the breathing gas inlet, the breathing gas outlet, the first outflow port, and the second outflow port may be formed by material of the body part. Fluid coupling between one or more of the breathing gas inlet, the breathing gas outlet, the first outflow port, and the second outflow port may be provided by material of the body part or by one or more other components coupled to the body part, such as a tube, hose or other coupling. Generally, the body part may be manufactured from various different materials, including metal, steel, aluminum, plastic material, resin-based material, reinforced material or others. Alternatively or additionally, the body part may comprise multiple members or parts, which may assembled, or it may be formed as single part. For example, at least a part of the body part and/or the respiration device may be manufactured from plastic material in a blowmolding process.

In the context of the present disclosure, the terms outlet and outlet port as well as inlet and inlet port may be interchangeably or synonymously used. The breathing gas inlet, the breathing gas outlet, the first outflow port, and the second outflow port may each comprise or define at least one opening, aperture or orifice for receiving and/or releasing breathing gas. The breathing gas inlet, the breathing gas outlet, the first outflow port, and the second outflow port may include or define physically separate openings. For instance, the breathing gas inlet, the breathing gas outlet, the first outflow port, and the second outflow port may be formed or arranged at different parts or portions of the body part. Further, at least a part or subset of the breathing gas inlet, the breathing gas outlet, the first outflow port, and the second outflow port may optionally be in fluid communication with one another.

As used herein, the CPAP mode and the PPV mode of the respiratory device may relate to different and distinct modes of operating or using the respiration device. Switching between the CPAP and PPV mode may be done based on or using the first outflow port. For example, the respiration device may be switched from the CPAP mode to the PPV mode based on at least partly occluding and/or closing the first outflow port and/or an opening thereof, such that the PPV pressure may be generated, which may exceed the CPAP or PEEP pressure. By removing the occlusion and/or releasing the first outflow port, the respiration device can be switched from the PPV mode into the CPAP mode.

The breathing gas supply may include one or more sources of breathing gas for supplying (fresh) breathing gas, for example an oxygen air mixture, to the breathing gas inlet. In particular, a substantially continuous flow of (fresh) breathing gas may be received by the breathing gas inlet from the breathing gas supply. The breathing gas supply may be coupled to the breathing gas inlet directly or by corresponding coupling means, for example including one or more tubes or hoses. At least a part of the breathing gas supply may be integrated or included in a ventilator, ventilation machine and/or resuscitator device. Alternatively, the breathing gas supply may be a standalone device.

The term patient interface may refer to or include any interface or means configured to establish a fluid communication between the breathing gas outlet and the patient, for example a patient's throat. Exemplary patient interfaces may include a nasal prong, a pair of nasal prongs, a mask or any other suitable device. The patient interface may be coupled directly or by corresponding coupling means to the breathing gas outlet, for example including one or more tubes or hoses.

As used herein, the patient may generally refer to an individual or subject to be ventilated or treated by CPAP and/or PPV treatment. In particular, the patient may refer to a newborn, child, infant and/or premature infant.

Further, the CPAP generator may refer to any device configured to generate the CPAP and/or PEEP pressure, for example at least at or near the breathing gas outlet of the body part, such that breathing gas or a flow of breathing gas can be supplied to the patient at said CPAP/PEEP pressure. Therein, the CPAP generator can be configured to generate the CPAP/PEEP pressure based on one or more physical principles, such as for example the Venturi effect (the smaller the cross-section, the greater the flow velocity), the Bernoulli principle (the higher the flow velocity, the lower the pressure), Coanda effect (an air stream in contact with a curved surface trying to follow it). The CPAP generator may be formed as one or more separate parts attached to and/or arranged at the body part. Alternatively or additionally, the CPAP generator may at least partly be arranged within the body part. Alternatively or additionally, the CPAP generator may at least partly be integrally formed with the body part. For instance, at least a portion or section of the body part may be configured and/or arranged as CPAP generator.

According to an embodiment, the first outflow port includes an opening for releasing, into an environment or surrounding of the respiration device, breathing gas received via the breathing gas inlet and/or exhaled by the patient and received via the breathing gas outlet. In particular, fresh and/or used breathing gas may be released into the environment of the respiration device in the CPAP mode, but also at least partly used or exhaled breathing gas supplied to the respiration device via the breathing gas outlet may be released through the first outflow port.

In an exemplary embodiment, the respiration device may be operated in the PPV mode based on at least partly occluding the first outflow port and/or at least one opening thereof. Alternatively or additionally, the respiration device may be operated in the CPAP mode based on opening the first outflow port and/or releasing at least one opening thereof. Alternatively or additionally, the respiration device may be switched from the PPV mode into the CPAP mode based on opening the first outflow port, and/or the respiration device may be switched from the CPAP mode into the PPV mode based on at least partly closing the first outflow port.

Alternatively or additionally, the respiration device may be switched from the PPV mode into the CPAP mode based on adjusting an outflow resistance of breathing gas through the first outflow port to a first resistance value, and the respiration device may be switched from the CPAP mode into the PPV mode based on adjusting the outflow resistance of breathing gas through the first outflow port to a second resistance value larger than the first resistance value. In other words, the respiration device may be switched between the CPAP and PPV mode based on adjusting, modifying and/or changing the outflow resistance of breathing gas through the first outflow port. Adjusting the outflow resistance may include adjusting and/or varying a diameter and/or cross-sectional area of the opening of the first outflow port.

As described in more detail hereinbelow, an adjustment of the outflow resistance of the first outflow port, for example opening and closing the port, may be performed manually, for example with a finger or by means of a manually actuatable closing element. This may allow for a simplified and fail-safe operation and handling of the respiration device by an operator or user. Also electronic or mechanical devices may be utilized for adjusting the outflow resistance of the first outflow port, for example for opening and closing the port.

As used herein, "in the PPV mode" may mean "when the respiration device is operated in and/or switched into the PPV mode for PPV treatment or ventilation". Alternatively or additionally, "in the CPAP mode" may mean "when the respiration device is operated in and/or switched into the CPAP mode for CPAP treatment or ventilation".

According to an embodiment, in the PPV mode of the respiration device, the ventilation pressure or PPV is generated solely or only using the flow of fresh breathing gas received via the breathing gas inlet. In other words, in the PPV mode, only the flow of breathing gas received via the breathing gas inlet can be supplied to the patient via the breathing gas outlet to inflate the patient's lungs and/or for mechanical ventilation. Accordingly, a single flow of breathing gas supplied via a single port, or the breathing gas inlet, can be used in the PPV mode for mechanical ventilation or PPV.

Alternatively or additionally, in the PPV mode of the respiration device, the ventilation pressure or PPV pressure may be generated solely or only based on stowing the flow of fresh breathing gas received via the breathing gas inlet within an interior volume and/or hollow compartment of the body part. For instance, an outflow resistance of the first outflow port may be increased in the PPV mode with respect to the CPAP mode. In particular, the first outflow port may be at least partly, for example entirely, occluded and/or closed in the PPV mode. The body part may at least partly be hollow and/or comprise one or more hollow compartments fluidly coupling the breathing gas inlet and the breathing gas outlet, and optionally one or both the first and second outflow ports, and configured to stow or dam the flow of fresh breathing gas from the breathing gas inlet, such that the ventilation pressure for PPV to mechanically inflate the lungs can be generated.

In yet another exemplary embodiment, both the ventilation pressure in the PPV mode and the positive end-expiratory pressure in the CPAP mode are generated solely, only and/or exclusively using the flow of (fresh) breathing gas received via the breathing gas inlet. In other words, no further flow of breathing gas other than the flow of breathing gas received via the breathing gas inlet may be used to generate both the CPAP or PEEP pressure in the CPAP mode and the ventilation pressure in the PPV mode of the respiration device. Accordingly, a single flow of breathing gas supplied or received via a single port, the breathing gas inlet, can be used to generate the PEEP in the CPAP mode and the ventilation pressure in the PPV mode of the respiration device. Hence, also a single source of breathing gas or a single breathing gas supply, for example of a ventilation machine or resuscitator device, can be utilized in both modes. This may save space at hospitals and operating theatres, simplify the connection of the device to other equipment, including the breathing gas supply, and simplify maintenance as well as handling.

According to an embodiment, in the PPV mode, the respiration device is configured to release excessive breathing gas from the body part via the second outflow port upon reaching or exceeding the ventilation pressure. Therein, excessive breathing gas or breathing gas having a pressure exceeding the ventilation pressure may be released via the expiration branch of a ventilator or ventilation machine couplable or coupled to the second outflow port. This configuration of the respiration device may avoid overinflation and over-breathing, and hence reduce or eliminate the risk for damage.

According to an embodiment, the ventilation pressure in the PPV mode is adjustable and/or controllable based on controlling the expiration valve and/or based on adjusting a release pressure of the expiration valve couplable to the second outflow port. The release pressure may denote an adjustable maximum pressure, upon which the expiration valve opens. The expiration valve may be couplable or coupled to the second outflow port directly, for example via a hose or tube, may be integrated in the second outflow port and/or may be part of an expiration branch or section of a ventilator or ventilation machine.

Generally, the ventilation pressure to inflate the patient's lungs may be patient-specific and may, among others, depend on the patient's anatomy. Hence, by providing an adjustable expiration valve, an optimum patient-specific ventilation pressure and overall ventilation may be achieved.

According to an embodiment, the expiration valve is an adjustable or controllable pop-off valve. In other words, the expiration valve can be a pressure-controlled valve, wherein a release pressure, at which the valve opens, can be adjusted or controlled, for example mechanically and/or electronically controlled. This may ensure that no overinflation of the patient's lungs can occur. Also volume-controlled valves may be used instead or in addition.

According to an embodiment, in the CPAP mode, the respiration device is configured to release excessive breathing gas from the body part via the first outflow port only. Therein, the term "excessive breathing gas" may refer to breathing gas received via the breathing gas inlet, but not inhaled by the patient, and/or breathing gas exhaled by the patient and received via the breathing gas outlet. For instance, the first outflow port may be substantially open or non-occluded in the CPAP mode, wherein an outflow resistance of the first outflow port may be adjusted or chosen, such that the CPAP or PEEP pressure is generated within at least a part of the body part, in particular at or near the breathing gas outlet.

According to an embodiment, the respiration device includes a single inlet port for receiving fresh breathing gas, which single inlet port is formed by the breathing gas inlet. Alternatively or additionally, the breathing gas inlet forms a single inlet port of the respiration device for receiving fresh breathing gas. Hence, also a single breathing gas supply can be used to generate the CPAP or PEEP pressure in the CPAP mode and the ventilation pressure in the PPV mode. Alternatively or additionally, the respiration device may include in total three outlets or outlet ports, which are the breathing gas outlet, the first outflow port and the second outflow port.

According to an embodiment, at least a part of the CPAP generator is integrally formed with the body part. Alternatively or additionally, the CPAP generator may be integrated in the body part. For example, the CPAP generator may be formed by a portion or section of the body part. Alternatively or additionally, the CPAP generator may be formed by material of the body part. Integration of the CPAP generator can allow for a simplified manufacturing of the respiration device. Also, handling of the respiration device may be simplified and robustness may be increased.

According to an embodiment, the body part includes one or more hollow compartments fluidly coupling the breathing gas inlet, the breathing gas outlet, the first outflow port and the second outflow port. Accordingly, at least a part of an interior volume of the body part may be hollow and may fluidly couple the breathing gas inlet and the breathing gas outlet, such that breathing gas received via the breathing gas inlet can be transferred through the body part, the one or more hollow compartments and/or the interior volume of the body part to the breathing gas outlet.

According to an embodiment, the body part includes a tubular middle section, wherein the breathing gas inlet and/or the second outflow port is laterally formed at the tubular middle section of the body part. The breathing gas inlet and the second outflow port may be formed at the same or different sides or portions of the tubular middle section.

Optionally, at least a part of the breathing gas inlet and/or at least a part of the second outflow port may laterally protrude from the tubular middle section of the body part. The breathing gas inlet and the second outflow port may protrude from the tubular middle section parallel to each other, in opposite directions or transverse to each other. Optionally, the breathing gas inlet and the second outflow port may be spaced apart from each along a longitudinal axis of the tubular middle section.

According to an embodiment, the breathing gas outlet and/or the first outflow port is formed at a face side and/or an end of the tubular middle section of the body part. For instance, the breathing gas outlet and the first outflow port may be formed at two opposing face sides of the tubular middle section of the body part.

It should be noted, that other designs and arrangements of the breathing gas outlet and/or the first outflow port, for example laterally at the tubular middle section of the body part, are encompassed by the present disclosure. Alternatively or additionally, the breathing gas inlet and/or the second outflow port may be arranged at a face side and/or an end of the tubular middle section.

According to an embodiment, the CPAP generator includes an adjustable discharge valve arranged at the first outflow port, wherein the discharge valve is configured to adjust a pressure of the breathing gas in the body part to the positive end-expiratory pressure for CPAP treatment in the CPAP mode. Based on the adjustable discharge valve, a patient-specific and optimum CPAP or PEEP pressure may be adjusted or set.

According to an embodiment, the discharge valve is manually adjustable or controllable. Alternatively or additionally, the discharge valve may include a manually displaceable closing element configured to at least partially occlude and/or close the first outflow port to generate the positive end-expiratory pressure for CPAP treatment. The manually displaceable element may be configured to adjust and/or control an outflow resistance and/or opening diameter of the first outflow port. The manually displaceable element may be displaced by an operator or user or by means of another device. The manually displaceable element may extend longitudinally or in transverse direction into the first outflow port, such that a cross-sectional area and/or opening diameter of the first outflow port, and hence a flow resistance of the first outflow port, can be controlled or adjusted.

In an exemplary embodiment, the manually displaceable closing element may include a knurled screw. Other means or devices, such as a displaceable bolt or pin, are also envisaged in the present disclosure.

According to an embodiment, the CPAP generator includes a Benveniste valve. Using a Benveniste valve may be particularly advantageous in that fluctuations in the CPAP/PEEP pressure may be reduced or minimized, thereby potentially reducing breathing work for the patient and increasing comfort. At least a part of or the entire Benveniste valve may be integrated in the body part, for example formed by material of the body part.

According to an embodiment, the Benveniste valve includes a tubular section fluidly coupled to the breathing gas inlet and configured to generate a jet of breathing gas within a first hollow compartment or first section of the body part. The first hollow compartment may fluidly couple the breathing gas inlet and the first outflow port. Optionally, the body part may include a second hollow compartment or second section physically separate from and arranged downstream of the first hollow compartment or section with respect to the flow of breathing gas received via the breathing gas inlet. The second hollow compartment may fluidly couple the second outflow port and the breathing gas outlet. Further optionally, the first hollow compartment or section and the second hollow compartment may be fluidly coupled via an opening, wherein the tubular section of the Benveniste valve may be formed to direct the jet of breathing gas onto the opening coupling and/or arranged between the first hollow compartment (or section) and the second hollow compartment (or section), such that the positive end-expiratory pressure for CPAP treatment can be generated downstream the opening and/or within the second hollow compartment or section of the body part.

According to an aspect of the present disclosure, there is provided a respiration system configured for positive pressure ventilation and continuous positive airway pressure treatment for neonatal resuscitation and respiratory support. The respiration system comprises at least one respiration device, as described hereinabove and hereinbelow, and at least one expiration valve fluidly coupled to the second outflow port of the at least one respiration device.

It is emphasized that, if technically sensible, any feature, function or element described herein with reference to the respiration device, equally applies to the respiration system, and vice versa.

According to an embodiment, the respiration system further includes a ventilation machine and/or ventilator for example for mechanical ventilation, wherein the at least one expiration valve is included in an expiratory branch of the ventilation machine. In other words, the expiration valve may be part of the ventilator or ventilation machine. Hence, a footprint and complexity of the respiration system can be reduced and equipment mostly present at hospitals can be utilized, thereby also saving costs. Alternatively or additionally, the respiration system may comprise a resuscitator device.

According to an embodiment, the respiration system further comprises at least one breathing gas supply fluidly coupled to the breathing gas inlet, preferably wherein the breathing gas supply includes a mixing device or mixer configured to adjust one or more of an oxygen concentration of the breathing gas, a flow rate of the breathing gas, a composition of the breathing gas, and an initial pressure of the breathing gas.

According to an embodiment, the respiration system further comprises at least one flow meter configured to determine at least one of a flow of breathing gas into the at least one respiration device and a flow of breathing gas out of the at least one respiration device. Also other elements or components may be included in the respiration system, such as a pressure sensor, one or more tubes or hoses and others.

Further aspects of the present disclosure may relate to use of a respiration device and/or system, as described hereinabove and hereinbelow, for neonatal resuscitation and/or respiratory support.

These and other aspects of the invention will be apparent from and elucidated with reference to the embodiments described hereinafter.

### Brief Description of the Drawings

- Fig. 1: schematically shows a respiration device according to an exemplary embodiment.
- Fig. 2: schematically shows a respiration system according to an exemplary embodiment.
- Fig. 3: schematically shows a respiration system according to an exemplary embodiment.
- Figs. 4A to 4C: each schematically illustrate operation of a respiration device according to an exemplary embodiment.
- Figs. 5A to 5C: each schematically illustrate a respiration device according to an exemplary embodiment.
- Figs. 6A to 6D: each schematically illustrate a respiration device according to an exemplary embodiment.

The Figures are schematic only and not true to scale. In principle, identical or like parts, and/or elements are provided with identical or like reference numerals in the Figures.

### Detailed Description of Exemplary Embodiments

Figure 1 schematically shows a respiration device 10 for positive pressure ventilation and continuous positive airway pressure treatment for neonatal resuscitation according to an exemplary embodiment.

The respiration device 10 comprises a body part 20, at which a breathing gas inlet 22, a breathing gas outlet 24, a first outflow port 26 and a second outflow port 28 are arranged. The body part 20 may be a substantially solid structure, for example manufactured from plastic material and/or metal. The body part 20 can have arbitrary shape, geometry and volume. For instance, the body part 20 may be substantially tubular, cylindrical, cubic, or cuboid. Other shapes and geometries are possible.

The breathing gas inlet 22 is fluidly couplable to a breathing gas supply 200 (see Figs. 2 and 3) for receiving a flow 23 of breathing gas, for example fresh breathing gas, from the breathing gas supply 200. The breathing gas outlet 24 is in fluid communication and/or fluidly coupled with the breathing gas inlet 22, such that at least a part of the flow 23 of breathing gas received via the breathing gas inlet 22 is transferred and/or conveyed through at least a portion of the body part 20 to the breathing gas outlet 24. Further, the breathing gas outlet 24 is fluidly couplable to a patient interface 510 (see Figs. 2 and 3), such as for example a mask 510, for supplying (fresh) breathing gas to a patient 500 (see Figs. 2 and 3). In particular, at least a part 25 of the flow 23 of breathing gas received at the breathing gas inlet 22 can be supplied to the patient 500 via the breathing gas outlet 24.

The first outflow port 26 is configured for releasing breathing gas from the body part 20, for example at least a part 27 of the flow 23 of breathing gas received via the inlet 22 or exhaled by the patient 500 and received via the breathing gas outlet 24. In particular, breathing gas may be released through the first outflow port 26 into an environment or surrounding of the respiration device 10. Hence, the first outflow port 26 may be fluidly couplable and/or coupled to the environment and/or surrounding of the respiration device 10.

The second outflow port 28 is configured for releasing breathing gas from the body part 20 via an expiration valve (see Figs. 2 to 4C). Further, the second outflow port 28 is fluidly couplable to one or both an expiration valve 32 and an expiratory branch of a mechanical ventilation machine 210b (see Fig. 3). The expiration valve 32 may be coupled to the second outflow port 28 directly or via some fluid coupling, such as a tube or hose, wherein the expiration valve 32 may optionally be part of a ventilation machine or ventilator 210b for mechanical ventilation. Alternatively or additionally, the expiration valve 32 may be integrated in the second outflow port 28 and/or the body part 20.

Any one or more of the breathing gas inlet 22, the breathing gas outlet 24, the first outflow port 26 and the second outflow port 28 can be arranged or fixedly attached as separate parts to the body part. Alternatively or additionally, any one or more of the breathing gas inlet 22, the breathing gas outlet 24, the first outflow port 26 and the second outflow port 28 can be integrally formed as single part with the body part 20. Accordingly one or more of the breathing gas inlet 22, the breathing gas outlet 24, the first outflow port 26 and the second outflow port 28 can be formed and/or defined by the body part 20 or material thereof.

Further, fluid communication between one or more of the breathing gas inlet 22, the breathing gas outlet 24, the first outflow port 26 and the second outflow port 28 can be provided by an interior volume of the body part 20 connecting one or more of the breathing gas inlet 22, the breathing gas outlet 24, the first outflow port 26 and the second outflow port 28. For instance, the body part 20 may include one or more channels or hollow compartments connecting one or more of the breathing gas inlet 22, the breathing gas outlet 24, the first outflow port 26 and the second outflow port 28. Alternatively or additionally, fluid communication between one or more of the breathing gas inlet 22, the breathing gas outlet 24, the first outflow port 26 and the second outflow port 28 can be provided by fluid couplings, such as one or more tubes or hoses.

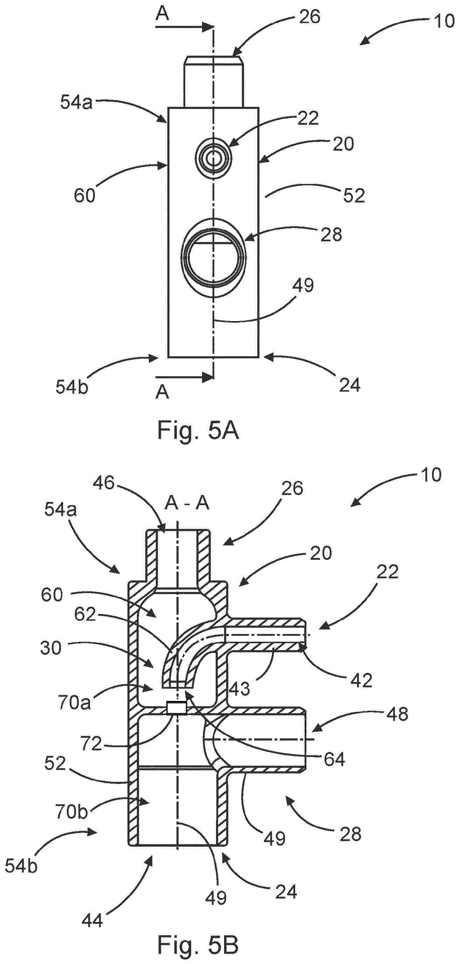

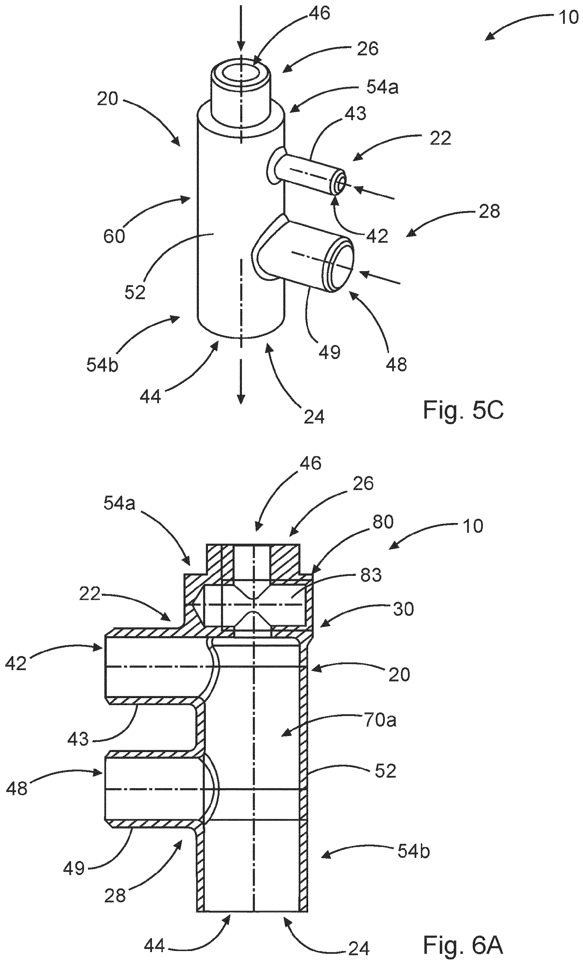

The respiration device 10 further comprises a continuous positive airway pressure, CPAP, generator 30 fluidly coupled between the breathing gas inlet 22 and the breathing gas outlet 24, and configured to generate a positive end-expiratory pressure, PEEP, for CPAP treatment or ventilation. Various designs of the CPAP generator 30 are envisaged in the present disclosure. Exemplary CPAP generators 30 are described in detail with reference to Figures 5A to 6D.

Generally, the CPAP generator 30 can be formed as separate part and arranged at or within the body part 20. Alternatively, the CPAP generator 30 can be integrally formed and/or formed as single part with the body part 20. For instance, the CPAP generator 30 may be formed by a portion or section of the body part 20. The CPAP generator 30 can be directly coupled, via a tube or hose, or via an interior volume of the body part to one or both the breathing gas inlet 22 and the breathing gas outlet 24.

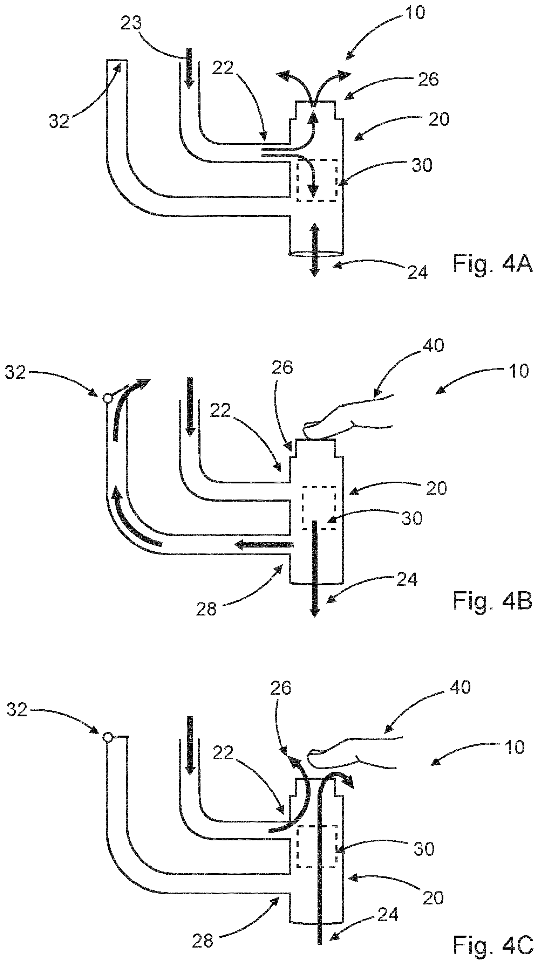

The respiration device 10, as will also be described in detail with reference to Figures 4A to 6D, is operable in two different operational modes, a CPAP mode for CPAP treatment and a PPV mode for PPV treatment.

In the CPAP mode, the first outflow port 26 is substantially open and/or non-occluded, such that breathing gas received via the breathing gas inlet 22 and/or exhaled breathing gas received via the breathing gas outlet 24 from the patient 500 can be released from an interior volume of the body part 20 through the first outflow port 26, for example into the environment or surrounding of the respiration device 10. In the CPAP mode, the CPAP generator 30 generates the substantially continuous PEEP, such that the patient's 500 lungs are kept open or partly inflated, even when the patient 500 fully exhales.

In the PPV mode, on the other hand, the first outflow port 26 is intermittently substantially closed and/or occluded, for example by means of a closing element and/or a finger of an operator or user. In particular, upon occluding the first outflow port 26, the flow 23 of breathing gas received via the breathing gas inlet 22 leads to an increase in pressure of the breathing gas within the body part 20. Upon reaching a ventilation pressure sufficient to mechanically inflate the patient's 500 lungs, at least a part 25 of the flow of breathing gas is supplied to the patient 500. Upon substantially fully inflating the patient's 500 lungs, the occlusion or closure of the first outflow port 26 can be removed, such that the pressure of the breathing gas drops and breathing gas can be exhaled by the patient 500 through the breathing gas outlet 24, the body part 20 and the first outflow port 26. To inflate the lungs, the first outflow port 26 can be occluded or closed again.

To prevent overinflation of the patient's 500 lungs in the PPV mode, the expiration valve 32 may be configured as pop-off valve 32 configured to open upon reaching a release pressure. The expiration valve 32 may be controllable and/or adjustable, based on adjusting the release pressure. Exemplary and non-limiting release pressures or ranges for the release pressure may be between 5 mbar and 100 mbar, for example between 10 mbar and 80 mbar, preferably between 20 mbar and 60 mbar, and further preferably between 30 mbar and below 60 mbar. For instance, a release pressure of 30 mbar may be used as a starting point and adjusted, this is increased or decreased, depending on the patient-specific respiration.

As described above, in the PPV mode of the respiration device 10, the ventilation pressure is generated solely using the flow 23 of fresh breathing gas received via the breathing gas inlet 22. In particular, the ventilation pressure for mechanically inflating the patient's 500 lungs is generated in PPV mode based on stowing the flow 23 of fresh breathing gas received via the breathing gas inlet 22 within an interior volume of the body part 20. For instance, an outflow resistance of the first outflow port 26 may be at least intermittently increased in the PPV mode with respect to the CPAP mode. Further, also in the CPAP mode, the CPAP pressure or PEEP is only generated using or based on the flow 23 of breathing gas received via the breathing gas inlet 22. No other source or flow of breathing gas may be required to generate any one of the ventilation pressure in the PPV mode and the CPAP/PEEP pressure in the CPAP mode.

Accordingly, the respiration device 10 according to the present disclosure advantageously allows connection or coupling to ventilators 210b or resuscitator device 210a (see Fig. 2), and can be operated both with manually controlled ventilation in the CPAP mode, as with a conventional T-piece, and ventilation- or machine-controlled by the ventilator 210b or resuscitator device 210a. This means that there is no need to switch between different ventilation devices, such as the single tube or hose resuscitator device 210a shown in Figure 2 and two-tube or hose ventilator machine 210b shown in Figure 3.

To allow for this, the respiration device 10 is configured to generate a continuous CPAP pressure, which may be manually adjustable. To perform positive pressure ventilation in the PPV mode, for example, the user can build up a peak pressure or ventilation pressure that is higher than the continuous CPAP pressure. This can be provided by the first outflow port 26, which can be intermittently occluded and/or closed, for example with a finger or other means. The occlusion or corresponding increase in outflow resistance through the first outflow port 26 can build up the positive pressure for inflating the patient's 500 lungs. For pure mechanical ventilation using a ventilator 210b, as described with reference to Figure 3, the first outflow port 26 can be completely closed, for example with a plug and/or closing element, and the release pressure of the expiration valve 32 can be chosen accordingly, such that excessive breathing gas can be released through the second outflow port 28.

For switching from manual to mechanical ventilation, the ventilator 210b may be correspondingly programmed, for example such that the expiration valve 32 is correctly controlled to remain open for a sufficient time period to deflate the patient's 500 lungs.

Figure 2 schematically shows a respiration system 100 with a respiration device 10 according to an exemplary embodiment. Unless stated otherwise, the respiration 100 system or device 10 of Figure 2 comprises the same features, elements and functions, as described with reference to Figure 1.

The respiration system 100 further includes a resuscitator device 210a, as can for example be used with conventional T-pieces. The resuscitator device 210a may provide or include the breathing gas supply 200. Generally, resuscitator devices 210a may also be referred to as single tube or hose respiration devices for neonatal resuscitation and initial respiratory support, as only (fresh) breathing gas is provided the system and exhaled breathing gas is released to the environment.

The resuscitator device 210a and/or breathing gas supply 200 of Figure 2 includes an oxygen connection 212 for receiving oxygen, a pressure 214 connection for receiving pressurized air and an oxygen-air mixer 216 for mixing oxygen and pressurized air to provide a flow 23 of breathing gas or fresh breathing gas. For instance, an outlet 218 of the mixer 216 may be coupled to the breathing gas inlet 22 of the respiration device 10 via a hose or tube 220. The breathing gas flows into the respiration device 10 via the breathing gas inlet 22 and can be delivered to the patient 500 via the breathing gas outlet 24, which may be coupled via a hose or tube to the patient interface 510, for example a mask 510. Alternatively, an endotracheal tube, through which the breathing gas is fed directly into the lungs, may be used.

The first outflow port 26 can be designed and configured as a valve to generate the PEEP pressure. At rest, the second outflow port 28 may be closed and the breathing gas can flow through the first outflow port 26, for example an opening or orifice thereof, into the environment or surrounding of the respiration device 10. Thereby, a continuous positive airway pressure (CPAP) and/or PEEP is generated by the CPAP generator 30, which may include the first outflow port 26 or at least part thereof. By increasing or decreasing the outflow orifice, an opening diameter and/or an outflow resistance of the first outflow port 26, this CPAP/PEEP pressure can be adjusted and or set. In the CPAP mode, when the patient 500 is breathing adequately spontaneously, it inhales the inflowing breathing gas and exhales it again via the first outflow port 26, whereby the pressure does not drop to zero even at the end of expiration (CPAP/PEEP).

To switch to the PPV mode, for example in the event of respiratory arrest, the first outflow port 26 on the respiration device 10 can be closed, for example with the finger or by other means. This can cause the pressure in the respiration device 10 or its body part 20 to rise rapidly, resulting in (manual) mechanical inspiration. To prevent the pressure from rising too much and potentially causing damage to the patient's lungs, for example the expiration valve 32, which may be a pressure release valve and/or pop-off valve 32, can be coupled to the second outflow port 28. Alternatively or additionally, a pressure relief valve 221 of the resuscitator device 210a can be opened, which may be located between the oxygen-air mixer 216 and the respiration device 10.

Figure 3 schematically shows a respiration system 100 with a respiration device 10 according to an exemplary embodiment. Unless stated otherwise, the respiration 100 system or device 10 of Figure 3 comprises the same features, elements and functions, as described with reference to Figures 1 and 2.

In contrast to the single tube resuscitator device 210a of Figure 2, Figure 3 shows a ventilation machine 210b or ventilator 210b for mechanical ventilation. Usually, such ventilators 210b have a closed hose or tube circuit, which is divided into two sections or branches, the inspiratory branch 250 for the supply of (fresh) breathing gas, and the expiratory branch 252 for the removal of (at least partly exhaled) breathing gas.

As shown in Figure 3, breathing gas may be supplied via an outlet 218 of the oxygen-air mixer 216 and a tube 220 to the breathing gas inlet 22 of the respiration device 10. The breathing gas outlet 24 may be coupled to the patient 500 via a patient interface 510, for example a mask 510 or endotracheal tube or tracheostoma. A continuous flow 23 of breathing gas coming from the machine 210b is provided, which flows through the inspiratory branch 250 to the respiration device 10 and through the expiratory branch 252 back to the ventilator 210b. For example, the second outflow port 28 may be coupled via a tube 221 to an expiration valve 32 of the ventilator 210b. Based on the expiration valve 32, for example the CPAP pressure throughout the tubing system can be controlled via the ventilation machine 210b.

An inspiration occurs when the expiratory valve 32 closes, which builds up the ventilation pressure. When the peak, maximum or release pressure of the expiration valve 32 is reached, the valve 32 opens again and the pressure is held for a short time and then falls back to the initial pressure or CPAP/PEEP level. This causes an expiration to occur.

The ventilation machine 210b and/or the resuscitator device 210a of Figure 2 may include further components, such as measuring and control systems. For instance, a pressure sensor 222 may monitor the pressure. Alternatively or additionally, one or more flow sensors 223 may monitor the flow 23 of fresh breathing gas and/or the flow 25 of breathing gas released through the second outflow port 28.

As described above, the respiration device 10 can be used with either of the resuscitator device 210a of Figure 2 and the ventilator 210b Figure 3. Hence, double equipment may not be necessary and the respiration device 10 can easily be switched from CPAP treatment to PPV treatment, for example in case of respiratory arrest, and switched back to the CPAP mode without changing equipment.

By providing the respiration device 10 with the breathing gas inlet 22, the breathing gas outlet 24, the first outflow port 26 and the second outflow port 28, an expiratory branch 225 of a ventilator 210b can be coupled to the second outflow port 28, wherein the expiration valve 32 should be arranged at the second outflow port 28 and/or the expiratory branch 252. The latter is typically arranged in the inspiratory branch 250 in conventional systems, which can therefore not be coupled to ventilation machines 210b or ventilators 210b.

As mentioned above, the ventilation machine 210b may be configured with dedicated software, for example providing a corresponding ventilation mode for use with the respiration device 10. The mode may ensure that the CPAP pressure may not be generated via the expiration valve 32 of the machine 210b, but via the first outflow port 28 of the respiration device 10. This may result in the breathing gas flowing into the respiration device 10 via the breathing gas inlet 22 at rest or in the CPAP mode and escaping into the environment via the first outflow 22 of the device 10.

Figures 4A to 4C each schematically illustrate operation of a respiration device 10 with a ventilator 210b, as described with reference to Figure 3. The respiration device 10 of Figures 4A to 4C may likewise be operated with the resuscitator device 210a of Figure 2. Unless stated otherwise, the respiration device 10 of Figures 4A to 4C comprises the same features, elements and functions, as described with reference to previous figures.

Figure 4A shows the respiration device 10 at rest or in the CPAP mode. The flow 23 of breathing gas is received via the breathing gas inlet 22 and excessive breathing gas can escape through the unconcluded and/or opened first outflow port 26 into the environment. Therein, the CPAP pressure is generated by the CPAP generator 30, which may for example include the first outflow port 26 to generate the CPAP/PEEP pressure based on adjusting an outflow resistance, opening diameter and/or opening of the port 26. The expiratory valve 32 of the machine 210a,b and/or coupled to the second outflow port 28 may be completely closed.

Figure 4B illustrates switching the device 10 into the PPV mode based on occluding or closing the first outflow port 26 with the finger 40. If the first outflow port 28 on the device 10 is closed, the ventilation pressure builds up and inspiration occurs. The pressure rises to the preset peak or release pressure of the expiration valve 32, upon which the expiration valve 32 in the ventilation machine 210b and/or coupled to the second outflow port 28 opens and the breathing gas flows into the environment or surrounding, when using a ventilator 210b via the expiratory branch 252 and when using a resuscitator device 210a directly via the second outflow port 28.

As soon as the first outflow opening 26 is released with the finger, as shown in Figure 4C, the pressure drops to the CPAP level, and the expiration valve 32 may close completely again. Hence, when using a ventilator 210b, there may be no flow in the expiratory branch 252. Further, the breathing gas flows out of the first outflow port 26 of the device 10 again, and the port 26 can be closed again for a further inhalation.

Figures 5A to 5C each schematically illustrate a respiration device 10 according to an exemplary embodiment. Unless stated otherwise, the respiration device 10 of Figures 5A to 5C comprises the same features, elements and functions, as described with reference to previous figures.

The body part 20 comprise a substantially tubular middle section 52. At a first end 54a or facial side 54a of the tubular middle section 52, the first outflow port 26 is arranged. At a second end 54b or facial side 54b, opposite to the first end 54a, of the tubular middle section 52, the breathing outlet 24 is arranged. Further, the breathing gas inlet 22 and the second outflow port 28 protrude from the tubular middle section 52. The inlet 22 and port 28 are exemplary arranged vertically above each other or spaced apart from each other in a direction parallel to a longitudinal axis 49 of the tubular middle section 52. The breathing gas inlet 22 and the second outflow port 28 may be arranged at same sides or different sides of the body part 20.

The breathing gas inlet 22 includes a port section 43 that protrudes from the tubular middle section 52 of the body part 20. The breathing gas inlet 22 and/or its port section 43 define an opening 42. An exemplary opening diameter may range from 2 mm to 7 mm, for example about 3 mm. Other diameters are possible.

The end 54b or facial side 54b of the tubular middle section 52 defines the breathing gas outlet 24 and/or an opening 44 of the breathing gas outlet 24. An exemplary opening diameter may range from 10 mm to 25 mm, for example about 15 mm. Other diameters are possible.

The end 54a or facial side 54a of the tubular middle section 52 defines the first outflow port 26 and/or an opening 46 thereof. An exemplary opening diameter may range from 5 mm to 15 mm, for example about 8 mm. Other diameters are possible.

The second outflow 28 includes a port section 49 that protrudes from the tubular middle section 52 of the body part 20. The second outflow 28 and/or its port section 49 define an opening 48. An exemplary opening diameter may range from 5 mm to 20 mm, for example about 10 mm. Other diameters are possible.

The respiration device 10 of Figures 5A to 5C or its CPAP generator 30 is based on or comprises a Benveniste valve 60. The CPAP generator 30 or Benveniste valve 60 includes a tubular section 62 fluidly coupled to the breathing gas inlet 22 and/or its opening 42, and configured to generate a jet of breathing gas within a first hollow compartment 70a of the body part 20. The respiration device 10 shown in Figures 5A to 5C can in particular be used with a two-tube ventilation system, for example the ventilator 210b or ventilation machine 210b described with reference to Figure 3.

The body part 20 includes a second hollow compartment 70b physically separate from and arranged downstream of the first hollow compartment 70a with respect to the flow of breathing gas received via the breathing gas inlet 22. The first hollow compartment 70a and the second hollow compartment 70b are fluidly coupled via an opening 72, wherein the tubular section 62 of the Benveniste valve is formed to direct the jet of breathing gas onto the opening 72 coupling the first hollow compartment 70a and the second hollow compartment 70b, such that the positive end-expiratory pressure for CPAP treatment is generated downstream the opening 72 and/or within the second hollow compartment 70b. As indicated in Figure 5B, the opening 72 can be formed as tubular section or element protruding towards tubular section 62 or an end 64 or nozzle 64 thereof.

Accordingly, the opening can be formed at a physical separation or barrier separating the first and second hollow compartments 70a, 70b, or the opening can be formed at an end of a tubular section protruding from the physical separation or barrier between the first and second hollow compartment 70a, 70b.

In the following, the exemplary embodiment with Beneviste valve 60 is summarized. In the Benveniste valve 60, a high flow of breathing gas may be accelerated from a flow source through the tubular section 62 to an end 64 or nozzle 64 of the tubular section 62, such that a jet of breathing gas that impinges onto the orifice or opening 72. The nozzle 64 may be arranged for example about 3 to 4 mm apart from the opening 72 and opposite the opening 72. The high flow creates a back pressure in the downstream areas of the respiration device 10. The pressure is then delivered to the child 500 or patient 500 as CPAP, for example, via a respiratory mask 510. Excessive breathing gas from the jet flows freely into the environment or surrounding via the first outflow port 26 in the CPAP mode.

The pressure in the CPAP mode or in the second hollow compartment 70b may be controlled by the level of the breathing gas flow that is supplied to the device 10 through the breathing gas inlet 22. In contrast to a conventional Benveniste valve, the excess breathing gas from the jet cannot escape freely into the environment, but must flow out of the second compartment 70b through the first hollow compartment 70a and through the first outflow port 26 or its opening 46.

If the opening 46 is closed with the finger, the device is switched to PPV mode and PPV ventilation, and breathing gas flowing in rapidly builds up a ventilation pressure in the entire device 10. To regulate this pressure, the second hollow compartment 70b is coupled to the second outflow port 28, to which an adjustable pressure relief valve 32 or expiration valve 32 can be attached. The expiration valve 32 may not be integrated in the breathing gas inlet 22. The breathing gas inlet 22 and the second outflow port 28 have thus physically separate openings 42, 48. The expiration valve 32 can be attached directly to the device 10 or connected to it via a breathing tube. The expiration valve 32 may also be integrated into the second outflow port 48, for instance in the protruding section 49.

Overall, this results in applicability for a normal two-tube system of a common neonatal ventilator 210b as shown in Figure 3. Via the inspiratory branch 52, the breathing gas is directed through the breathing gas inlet 22 to the nozzle 64 and the created jet generates the CPAP pressure, which is controllable by the breathing gas flow 23 into the device 10. The second outflow port 28 connects the second hollow compartment 70b to the expiration valve 32 of the ventilator 210b via the expiratory branch 52. This may allow the peak or ventilation pressure pressure to be regulated. The expiration valve 32 may be closed as long as the first outflow port 26 is open and the CPAP pressure is generated only via the flow through the Benveniste valve 60 or CPAP generator 30. When the breathing gas cannot flow out freely due to closure of the first outflow port 26, the PPV pressure is generated and can be regulated via the expiration valve 32.

If mechanical ventilation is switched to in the PPV mode, the first outflow port 26 can be closed with a plug or other suitable device. This enables mechanical ventilation without changing the machine and hose system.

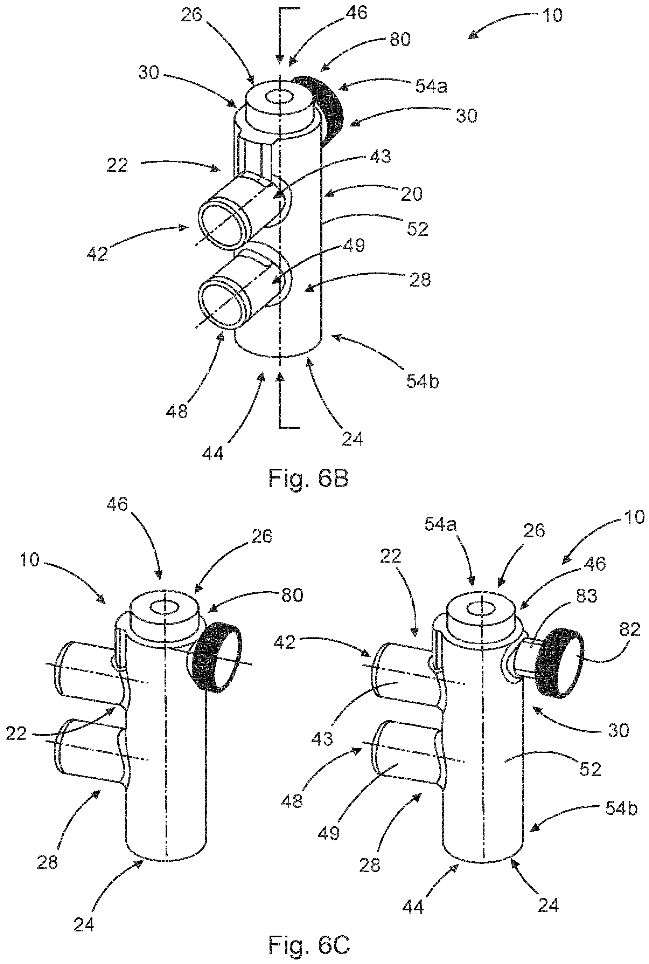

Figures 6A to 6D each schematically illustrate a respiration device 10 according to an exemplary embodiment. Unless stated otherwise, the respiration device 10 of Figures 6A to 6D comprises the same features, elements and functions, as described with reference to previous figures.

The breathing gas inlet 22 includes a port section 43 that protrudes from the tubular middle section 52 of the body part 20. The breathing gas inlet 22 and/or its port section 43 define an opening 42. An exemplary opening diameter may range from 5 mm to 15 mm, for example about 10 mm. Other diameters are possible.

The end 54b or facial side 54b of the tubular middle section 52 defines the breathing gas outlet 24 and/or an opening 44 of the breathing gas outlet 24. An exemplary opening diameter may range from 10 mm to 25 mm, for example about 15 mm. Other diameters are possible.

The end 54a or facial side 54a of the tubular middle section 52 defines the first outflow port 26 and/or an opening 46 thereof. An exemplary opening diameter may range from 5 mm to 15 mm, for example about 6 mm. Other diameters are possible.

The second outflow 28 includes a port section 49 that protrudes from the tubular middle section 52 of the body part 20. The second outflow 28 and/or its port section 49 define an opening 48. An exemplary opening diameter may range from 5 mm to 15 mm, for example about 10 mm. Other diameters are possible.

In contrast to the embodiment shown in Figures 5A to 5C, the CPAP generator 30 of the device 10 of Figures 6A to 6D is based on creating the CPAP/PEEP pressure only based on the opening 46 of the second outflow port 26. For this purpose, the CPAP generator 30 includes an adjustable discharge valve 80 arranged at the first outflow port 26, wherein the discharge valve 80 is configured to adjust a pressure of the breathing gas in the body part 20 to the positive end-expiratory pressure for CPAP treatment in the CPAP mode, e.g. when the first outflow port 26 is non-occluded and/or open. In particular, the discharge valve 80 may be manually adjustable or controllable. For example, the discharge valve 80 may include a manually displaceable closing element 83 configured to at least partially occlude the first outflow port 26 and/or its opening 46 to generate, adjust and/or control the positive end-expiratory pressure for CPAP treatment in the CPAP mode.

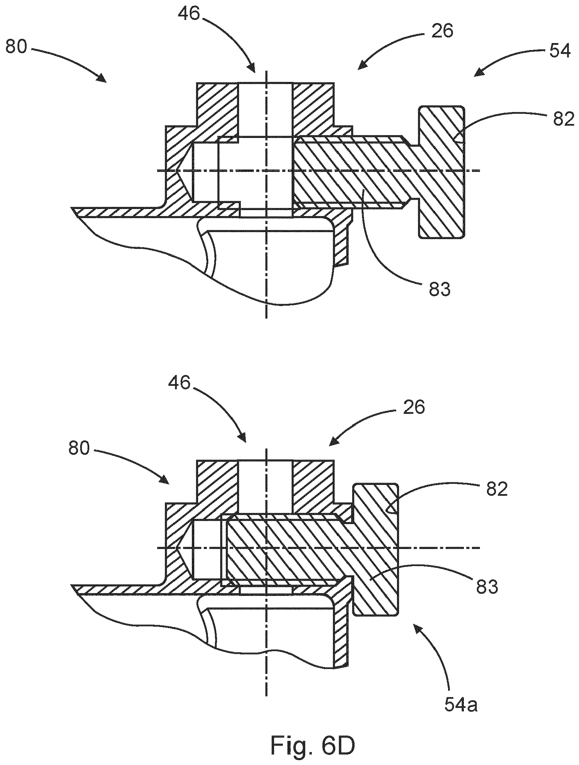

As shown in Figures 6A, 6C and 6D, the closing element 83 may protrude laterally, for example orthogonal or transverse to the longitudinal axis of the tubular middle section 52 of the body part, into the first outflow port 26. Depending on its position, an opening diameter and/or outflow resistance of the first outflow port 26 may be controlled and/or adjusted. In the example shown in Figures 6A to 6D, the closing element 83 can be displaced by or includes a knurled screw 82. Other designs are possible.

Breathing gas is introduced into respiration device 10 via either the breathing gas inlet 22 or the second outflow port 28. Both openings may be designed as possible inspiratory or expiratory openings and may not create any relevant additional flow resistance. If the breathing gas is supplied via the second outflow port 28, then the expiration valve 32 may be attached to the breathing gas inlet 22, and vice versa.

During manual or mechanical ventilation in the PPV mode, the breathing gas may flow out of the respiration device 10 via the first outflow port 26 into the environment or surrounding. Depending on the setting of the discharge valve 80 at this point, a constant CPAP pressure can be created. This can be changed manually based on actuating or controlling the discharge valve 80. The expiration valve 32 may be closed in the CPAP mode.

If the user closes the first outflow port 26, for example with a finger during manual ventilation, an overpressure builds up device 10 or body part 20. This can be regulated by the expiration valve 32. As soon as the pressure exceeds the peak, maximum or release pressure, it opens and the excess breathing gas flows out through it. If the user releases the first outflow port 26, the pressure drops to the CPAP level and the expiration valve 32 closes. The breathing gas flows back into the environment via the first outflow port 26 until it is closed again.

When switching to mechanical ventilation in the PPV mode, the first outflow port 26 may be closed completely. This can be done by the knurled screw 82 or by a plug or by some other type of mechanism.

The respiration device 10 shown in Figures 6A to 6D can in particular be used with a single tube resuscitator device 210a, for example as described with reference to Figure 2, wherein the opening 28 may be closed with a plug or other means. When using the respiration device 10 of Figures 6A to 6D with a two-tube ventilation system, for example the ventilator 210b or ventilation machine 210b described with reference to Figure 3, opening 28 may be connected to the ventilator 210b.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A respiration device (10) for positive pressure ventilation and continuous positive airway pressure treatment for neonatal resuscitation, the respiration device (10) comprising:
a body part (20) comprising a) a breathing gas inlet (22) fluidly couplable to a breathing gas supply (200) for receiving a flow of breathing gas, b) a breathing gas outlet (24) in fluid communication with the breathing gas inlet (22) and fluidly couplable to a patient interface (510) for supplying breathing gas to a patient (500), c) a first outflow port (26) for releasing breathing gas from the body part (22), the first outflow port (26) being fluidly couplable to a surrounding of the respiration device (10), and d) a second outflow port (28) for releasing breathing gas from the body part (20), the second outflow port being fluidly couplable to one or both an expiration valve (32) and an expiratory branch (52) of a ventilation machine (210b); and
a continuous positive airway pressure, CPAP, generator (30) fluidly coupled between the breathing gas inlet (22) and the breathing gas outlet (24), and configured to generate a positive end-expiratory pressure for CPAP treatment;
wherein the respiration device (10) is operable in a CPAP mode for CPAP treatment based on releasing breathing gas from the body part (20) to the surrounding of the respiration device (10) via the first outflow port (26), such that the positive end-expiratory pressure is generated within the body part (20); and
wherein the respiratory device (10) is operable in a positive pressure ventilation, PPV, mode for PPV treatment based on at least intermittently occluding the first outflow port (26), such that a ventilation pressure for PPV treatment is generated within the body part (20).

2. The respiration device (10) according to claim 1, wherein, in the PPV mode of the respiration device, the ventilation pressure is generated solely using a flow (23) of fresh breathing gas received via the breathing gas inlet (22); and/or
wherein, in the PPV mode of the respiration device, the ventilation pressure is generated solely based on stowing a flow (23) of fresh breathing gas received via the breathing gas inlet (22) within an interior volume of the body part.

3. The respiration device (10) according to any one of the preceding claims, wherein both the ventilation pressure in the PPV mode and the positive end-expiratory pressure in the CPAP mode are generated solely using a flow (23) of fresh breathing gas received via the breathing gas inlet (22).

4. The respiration device (10) according to any one of the preceding claims, wherein, in the PPV mode, the respiration device is configured to release excessive breathing gas from the body part (20) via the second outflow port (28) upon reaching or exceeding the ventilation pressure.

5. The respiration device (10) according to any one of the preceding claims, wherein, in the CPAP mode, the respiration device is configured to release excessive breathing gas from the body part (20) via the first outflow port (26) only.

6. The respiration device (10) according to any one of the preceding claims, wherein the ventilation pressure in the PPV mode is adjustable based on controlling the expiration valve (32) and/or based on adjusting a release pressure of the expiration valve (32) couplable to the second outflow port (28), preferably wherein the expiration valve (32) is an adjustable or controllable pop-off valve.

7. The respiration device (10) according to any one of the preceding claims,
wherein the respiration device includes a single inlet port (22) for receiving fresh breathing gas, which single inlet port is formed by the breathing gas inlet (22); and/or
wherein the breathing gas inlet (22) forms a single inlet port (22) of the respiration device (20) for receiving fresh breathing gas.

8. The respiration device (10) according to any one of the preceding claims, wherein at least a part of the CPAP generator (30) is integrally formed with and/or integrated in the body part (20).

9. The respiration device (10) according to any one of the preceding claims, wherein the body part (20) includes one or more hollow compartments (70a, 70b) fluidly coupling the breathing gas inlet (22), the breathing gas outlet (24), the first outflow port (26) and the second outflow port (28).

10. The respiration device (10) according to any one of the preceding claims, wherein the body part (20) includes a tubular middle section (52), and
wherein the breathing gas inlet (22) and/or the second outflow port (28) is laterally formed at the tubular middle section (52) of the body part (20); preferably wherein at least a part of the breathing gas inlet (22) and/or at least a part of the second outflow port (28) laterally protrudes from the tubular middle section (52) of the body part (20).

11. The respiration device (10) according to claim 10, wherein the breathing gas outlet (24) and/or the first outflow port (26) is formed at a face side (54a,b) of the tubular middle section (52) of the body part (20), preferably wherein the breathing gas outlet (24) and the first outflow port (28) are formed at two opposing face sides (54a,b) of the tubular middle section (52) of the body part (20).

12. The respiration device (10) according to any one of the preceding claims,
wherein the CPAP generator (30) includes an adjustable discharge valve (80) arranged at the first outflow port (26), and
wherein the discharge valve (80) is configured to adjust a pressure of the breathing gas in the body part (20) to the positive end-expiratory pressure for CPAP treatment in the CPAP mode, preferably wherein the discharge valve (80) is manually adjustable and/or preferably wherein the discharge valve (80) includes a manually displaceable closing element (83) configured to at least partially occlude the first outflow port (26) to generate the positive end-expiratory pressure for CPAP treatment.

13. The respiration device (10) according to any one of the preceding claims,
wherein the CPAP generator (30) includes a Benveniste valve (60).

14. The respiration device (10) according to claim 13, wherein the Benveniste valve (60) includes a tubular section (62) fluidly coupled to the breathing gas inlet (22) and configured to generate a jet of breathing gas within a first hollow compartment (70a) of the body part (20);
wherein the body part includes a second hollow compartment (70b) physically separate from and arranged downstream of the first hollow compartment (70a) with respect to the flow of breathing gas received via the breathing gas inlet (22);
wherein the first hollow compartment (70a) and the second hollow compartment (70b) are fluidly coupled via an opening (72); and
wherein the tubular section (62) of the Benveniste valve (60) is formed to direct the jet of breathing gas onto the opening (72) coupling the first hollow compartment (70a) and the second hollow compartment (70b), such that the positive end-expiratory pressure for CPAP treatment is generated downstream the opening (72) and/or within the second hollow compartment (70b).

15. A respiration system (100) configured for positive pressure ventilation and continuous positive airway pressure treatment for neonatal resuscitation, the respiration system comprising:
at least one respiration device (10) according to any one of the preceding claims; and
at least one expiration valve (32) fluidly coupled to the second outflow port (28) of the at least one respiration device (10); and
optionally one or more of a ventilation machine (210b), wherein the at least one expiration valve is included in an expiratory branch (52) of the ventilation machine (210b) and a resuscitator device (210a).
